# FASCICULE DE BREVET EUROPEEN

(11) **EP 2 437 698 B1**
(45) Date de publication et mention de la délivrance du brevet: **06.01.2016**
(21) Numéro de dépôt: 10728830.0
(22) Date de dépôt: 27.05.2010
(51) Int. Cl.: A61F 9/00, A61M 35/00, B05B 11/00, B65D 83/14, B65D 83/16

(54) **PULVÉRISATEUR ÉQUIPÉ D'UN DISPOSITIF D'ASSISTANCE À LA DÉLIVRANCE DE GOUTTES D'UN LIQUIDE CONTENU DANS LEDIT PULVÉRISATEUR**
SPRAY MIT HILFSVORRICHTUNG FÜR DIE AUSGABE VON FLÜSSIGKEITSTROPFEN IN DIESEM SPRAY
SPRAYER PROVIDED WITH A DEVICE THAT ASSISTS IN SUPPLYING DROPS OF A LIQUID CONTAINED IN SAID SPRAYER

(30) Priorité: 03.06.2009 FR 0953649
(43) Date de publication de la demande: 11.04.2012
(73) Titulaire: Laboratoires THEA, 63100 Clermont-Ferrand (FR)
(72) Inventeur: FAURIE, Michel, F-63960 Veyre-monton (FR)
(74) Mandataire: Cabinet Laurent & Charras
(86) Numéro de dépôt international: PCT/FR2010/051022
(87) Numéro de publication internationale: WO 2010/139883

(56) Documents cités:
- EP-A- 0 335 513
- WO-A-98/30469
- DE-A1-102005 004 889
- US-A- 5 588 564
- US-B1- 6 276 568

## Description

### DOMAINE TECHNIQUE

L'invention se rattache au domaine des dispositifs utilisés pour délivrer des gouttes de liquide à partir d'un réservoir utilisant une pompe mécanique. Elle vise plus spécifiquement un dispositif d'aide permettant d'améliorer la précision de cette délivrance et d'ergonomie des opérations. Elle trouve une application toute particulière dans le domaine de l'instillation oculaire, sans toutefois y être totalement limitée.

Dans la suite de la description, l'invention sera donc plus spécifiquement décrite pour cette application, la transposition à d'autres domaines d'application pouvant se faire sans difficulté majeure.

### TECHNIQUES ANTERIEURES

Dans le domaine des soins oculaires au sens large, il est fréquemment nécessaire d'instiller des gouttes d'un liquide, qu'il s'agisse d'un collyre ou d'autres substances, directement à la surface de l'oeil. A ce jour, les conditionnements habituels présentent une ergonomie qui n'est pas toujours optimale, ou alors qui n'est pas compatible avec les nouveaux dispositifs utilisés pour la délivrance de solutions ophtalmiques formulées sans conservateur.

En effet, dans le cas le plus courant, les collyres sont conditionnés dans des flacons souples, permettant de délivrer des gouttes par pression des doigts sur les parois déformables d'un flacon qui, présentant une mémoire de forme, permet des instillations subséquentes dans les mêmes conditions que la distribution initiale. On connaît également les conditionnements unidose, réalisés en un matériau plastique déformable et qui contiennent une certaine quantité de gaz qui joue le rôle de transmetteur de force pour permettre, lorsque le conditionnement est en position verticale avec un embout proche de l'oeil, l'expulsion de la goutte se trouvant en partie basse du conditionnement.
Avec l'avènement des produits dits « sans conservateur », d'autres moyens d'obtention des gouttes ont été imaginés.

C'est ainsi qu'ont été proposés des conditionnements plus élaborés, permettant d'utiliser des solutions liquides exemptes de conservateur. Plus précisément, ces conditionnements comportent une membrane antimicrobienne hydrophobe qui sépare la réserve de liquide de l'embout distributeur de goutte, qui communique avec le milieu extérieur. Mécaniquement, le réservoir est muni de soufflets qui, sous l'effet de la pression des doigts, exercée dans l'axe du réservoir, crée une pression propre à faciliter l'expulsion des gouttes au travers de ladite membrane antimicrobienne. L'utilisation des soufflets permet de compenser la dépression équivalente au volume de solution délivrée, car la membrane hydrophobe n'accepte pas le retour de l'air.

D'autres dispositifs sont apparus pour pouvoir contenir des collyres sans conservateur et fonctionnant sans l'emploi de membranes antimicrobiennes. Ainsi, on connaît des conditionnements du type « pulvérisateur », qui comportent une pompe mécanique étudiée pour distribuer des gouttes, actionnée par le mouvement imposé à une partie mobile du pulvérisateur. Le principe de tels pulvérisateurs a été décrit dans le document FR-2 739 294, dans une application à la pulvérisation nasale.

Pour pouvoir sécuriser une étanchéité vis-à-vis des microorganismes ambiants, ce dispositif nécessite une force de retour de l'ordre de quelques Newtons au niveau du chemin hydraulique du liquide à distribuer. Une pression axiale contrariant cette force de retour doit être appliquée entre l'embout de sortie du pulvérisateur et le corps du système pour pouvoir expulser les gouttes à distribuer.

On conçoit que l'ergonomie de ce type de dispositif n'est pas optimale pour l'instillation oculaire.

En effet, le mode opératoire pour la distribution de gouttes au moyen de ce type de dispositif est le suivant. L'index et le majeur sont généralement disposés de part et d'autre de l'embout, au niveau de la zone mobile du pulvérisateur. Le pouce est quant à lui disposé sous le fond du pulvérisateur, de sorte que pour présenter l'embout devant l'oeil, il est nécessaire d'effectuer un mouvement du bras vers le haut et une rotation importante du poignet. Ce type de mouvement en extension n'est pas toujours compatible avec une bonne précision de positionnement. De plus, dans cette configuration, l'index et le majeur se trouvent à proximité directe de l'oeil, et empêchent ainsi de visualiser clairement l'opération avec l'autre oeil lorsqu'elle est effectuée devant un miroir. Un autre mode opératoire est proposé par les fabricants de tels pulvérisateurs, qui prévoit la tenue du dispositif entre le pouce et l'index ; l'index appliquant sur le fond du récipient la force en réaction du pouce qui comprime la partie mobile de la pompe. Quel que soit le geste préconisé, il est parfois difficile d'exercer les efforts appropriés avec un bon niveau de précision lorsque, comme c'est le cas avec ce type de pulvérisateur, ces efforts sont exercés au niveau des dernières phalanges des doigts qui viennent au contact du pulvérisateur. Dans certains cas, il est possible que les mouvements exercés sur le pulvérisateur provoquent le déplacement de ce dernier jusqu'à venir au contact de l'oeil, ce qui est bien entendu préjudiciable.

Le document WO 98/30469 décrit un pulvérisateur équipé d'un dispositif d'assistance de délivrance de gouttes. Le dispositif comprend un récipient muni d'une poignée recevant le pulvérisateur et un couvercle venant se visser sur le récipient. Le couvercle est muni d'une poignée montée en rotation, et venant appuyer sur la tête du pulvérisateur.

Les documents US 6276548 B1 et DE 10 2005 004 889 A1 décrivent un dispositif d'assistance de délivrance de gouttes venant s'insérer sur l'embout de sortie du pulvérisateur, le dispositif étant muni de deux ailettes latérales.

Le document EP 335 513 décrit un dispositif d'assistance sous forme d'une pince venant exercer une pression sur le corps du flacon.

Le document US 5588 564 décrit enfin un adaptateur oculaire venant s'insérer à l'extrémité du pulvérisateur.

L'invention cherche donc à pallier ces différents inconvénients, en cherchant donc à obtenir un dispositif qui combine à la fois ergonomie et précision.

### EXPOSE DE L'INVENTION

L'invention concerne donc un dispositif d'assistance à la délivrance de gouttes d'un liquide contenu dans un pulvérisateur. Ce pulvérisateur est du type comprenant une portion mobile formant une pompe mécanique, elle-même équipée d'un embout de sortie.

Conformément à l'invention, ce dispositif se caractérise en ce qu'il comprend :
- une première partie agencée pour recevoir le fond du pulvérisateur ;
- une deuxième partie, apte à coopérer avec cette portion mobile, en laissant apparent l'embout de sortie.

Chacune des parties comporte une ailette d'appui latérale. Les deux ailettes sont sensiblement en regard l'une de l'autre pour permettre un mouvement de la portion mobile du pulvérisateur, et donc un actionnement de la pompe, lorsqu'une force est exercée entre les deux ailettes.

Autrement dit, le pulvérisateur se trouve ainsi équipé d'une ailette qui est décalée latéralement de l'embout de sortie du pulvérisateur, ce qui libère le voisinage de l'embout venant à proximité de l'oeil. Le reste du pulvérisateur reçoit donc une autre ailette, disposée dans le même plan que la première ailette d'appui pour permettre, lorsque les deux ailettes se rapprochent, qu'une pression soit exercée sur la pompe mécanique, provoquant ainsi l'expulsion des gouttes.

En d'autres termes, l'invention consiste à équiper le pulvérisateur d'un mécanisme qui permet d'agir sur la pompe mécanique en écartant les doigts de l'embout du pulvérisateur d'une part, et en permettant une préhension plus efficace d'autre part. Ainsi, dans le cas de l'application oculaire, les doigts qui appliquent la pression nécessaire à la délivrance des gouttes, se trouvent décalés de l'axe du pulvérisateur, et donc éloignés de l'oeil. De cette manière, il est possible d'agir sur les ailettes d'appui avec la deuxième, voire la première phalange des doigts, avec un niveau d'effort et une précision bien meilleurs que dans les systèmes existants pour lesquels la pression est exercée avec les dernières phalanges.

Dans une forme avantageuse de réalisation, les deux parties du dispositif présentent une zone de recouvrement partielle qui autorise ainsi le coulissement relatif des deux parties l'une par rapport à l'autre, en aidant le guidage axial perturbé par le décalage de la force d'appui.

Avantageusement, en pratique, le dispositif peut comporter, au niveau de la zone de recouvrement de la première et deuxième parties, des moyens d'indexation permettant de bloquer la rotation d'une partie par rapport à l'autre, et de conserver ainsi un alignement des deux ailettes.

Selon une autre caractéristique de l'invention, l'une ou l'autre des parties recevant le pulvérisateur, ou bien encore les deux, peuvent posséder des excroissances présentes à l'intérieur du corps recevant le pulvérisateur, de manière à bloquer le mouvement de ce dernier par rapport à l'une ou l'autre des parties. On empêche ainsi que l'un ou l'autre des éléments du dispositif ne se sépare inopinément du pulvérisateur.

Ainsi, dans une première forme de réalisation, la première partie, c'est-à-dire celle recevant le fond du pulvérisateur, peut comporter une nervure disposée dans une région du volume qui reçoit le fond du pulvérisateur, et qui est agencée pour venir au contact de ce dernier, afin d'en bloquer le mouvement. Ceci est d'autant plus efficace que le matériau qui forme la réserve du pulvérisateur est compressible, et donc est introduit en force dans la première partie du dispositif caractéristique.

Avantageusement, en pratique, la seconde partie, c'est-à-dire celle qui vient coiffer la zone d'actionnement de la pompe, peut présenter des moyens de solidarisation, par encliquetage par exemple, sur la partie mobile du pulvérisateur.

Pour améliorer l'ergonomie du dispositif, on peut prévoir qu'au moins une des ailettes présente une forme bombée dont la courbure est orientée vers l'ailette opposée. Autrement dit, l'une des ailettes, voire les deux, peuvent épouser une forme globale en tuile tel qu'elle épouse la courbure des doigts venant la presser, sans former d'arête saillante.

En pratique, les ailettes peuvent également être renforcées par la présence d'au moins une nervure reliant la face de l'ailette orientée vers l'autre ailette, et le corps de la partie sur lequel elle est implantée. Autrement dit, la déformation de l'ailette est limitée par la présence de nervures qui s'opposent à sa flexion.

Le dispositif conforme à l'invention peut être mis en place sur le flacon lors de sa fabrication, et plus particulièrement après l'opération de remplissage. L'étiquetage peut alors être mis en place directement sur l'une ou l'autre des parties du dispositif caractéristique.

Il est également possible de prévoir que l'une ou l'autre des parties, et en particulier celle qui recouvre la partie principale du corps du réservoir, présente une ouverture permettant de visualiser l'étiquetage du flacon, voire même la mise en place sur un flacon existant. Dans ce cas, on privilégiera l'emploi à l'intérieur de l'une des parties de moyens de blocage compressibles, permettant de s'adapter à différentes sections du réservoir.

### DESCRIPTION SOMMAIRE DES FIGURES

La manière de réaliser l'invention, ainsi que les avantages qui en découlent, ressortiront bien de la description du mode de réalisation qui suit, à l'appui des figures annexées dans lesquelles :
La figure 1 est une vue de côté d'un dispositif conforme à l'invention, montré avant montage sur un pulvérisateur.
Les figures 2 et 3 sont des vues en perspective sommaire respectivement des première et deuxième parties du dispositif de la figure 1.
La figure 4 est une vue en perspective sommaire montrant un pulvérisateur équipé du dispositif selon l'invention, après assemblage.
Les figures 5 et 6 sont des vues en coupe longitudinale selon les plans V et VI de la figure 4.
La figure 7 est une vue en perspective sommaire d'une variante de réalisation de l'invention.
La figure 8 est une vue en coupe longitudinale d'une autre variante de réalisation.

### MANIERE DE REALISER L'INVENTION

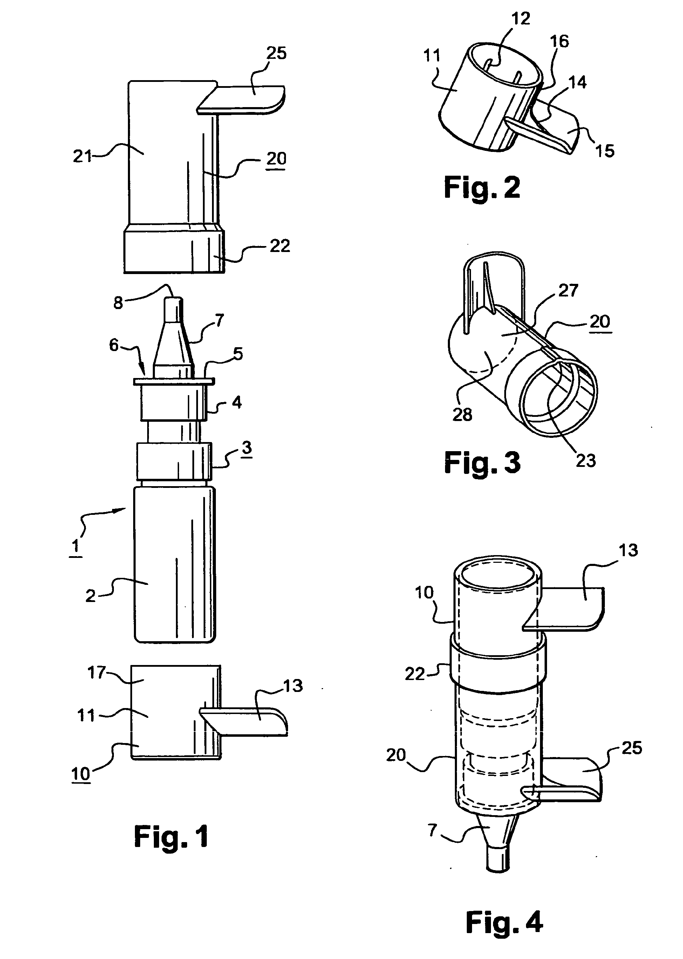

Comme illustré à la figure 1, le dispositif conforme à l'invention est destiné à s'adapter à un pulvérisateur **1** qui comporte un corps principal **2** formant le réservoir de liquide, qui dans la forme illustrée, est de géométrie sensiblement cylindrique.

Dans sa partie supérieure, ce corps **2** est équipé de la pompe mécanique **3** qui comporte la partie mobile **4**, apte à se déplacer axialement en direction du réservoir **2**. Cette partie mobile présente une collerette **5** de plus grand diamètre, définissant la surface **6** orientée vers le haut du pulvérisateur, et sur lequel viendra prendre appui le dispositif caractéristique. Cette collerette se prolonge par l'embout de sortie **7** présentant l'orifice **8** par lequel les gouttes sont délivrées.

Conformément à l'invention, le dispositif d'aide à la délivrance de gouttes se compose de deux parties, à savoir une première partie **10** recevant le fond du distributeur et une deuxième partie **20** recouvrant la partie supérieure du pulvérisateur.

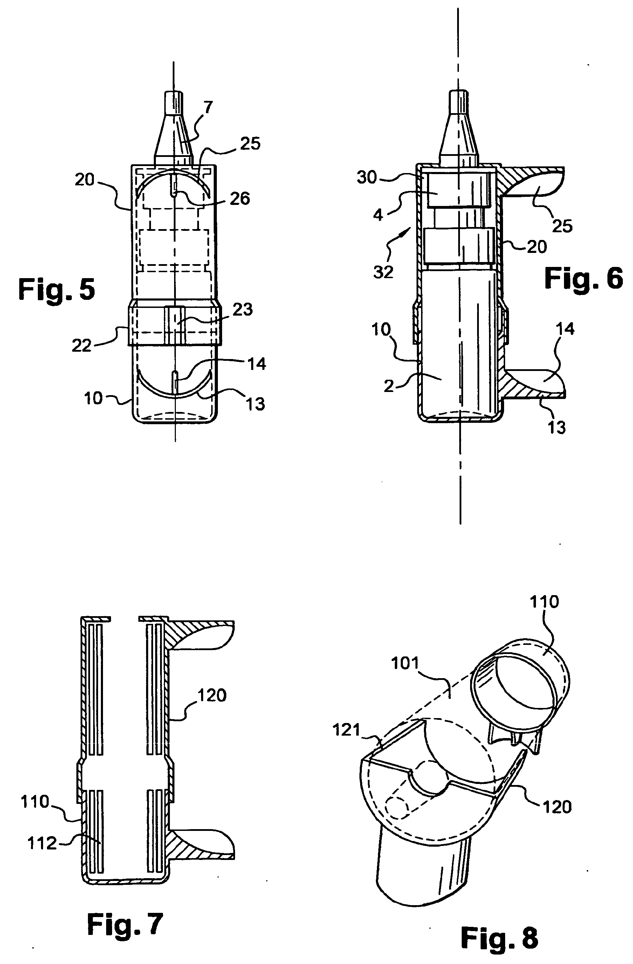

La première partie **10** possède un corps principal **11** de forme générale cylindrique, d'un diamètre interne légèrement supérieur au diamètre du réservoir **2**. Comme illustré à la figure 2, le corps cylindrique **11** possède sur sa face interne des différentes nervures **12** permettant le blocage du réservoir **2** lorsque celui-ci est introduit dans le volume l'accueillant. La première partie **10** possède, conformément à l'invention, une ailette d'appui latérale **13** rapportée sur la face extérieure du corps **11**. Plus précisément, cette ailette est incurvée et présente une forme générale en tuile, avec une courbure orientée vers le haut du pulvérisateur. La jonction entre la tuile **13** et le corps **11**, du fait de l'incurvation de l'ailette **13**, confère une certaine rigidité, qui est encore améliorée par la présence de nervures **14** reliant la face supérieure **15** de l'ailette **13** et le côté extérieur du corps **11**. Dans la forme illustrée, on observe que la nervure **14** se prolonge le long du corps **11** pour former une excroissance **16** qui coopère avec des moyens complémentaires **23** présents sur la deuxième partie **20** pour assurer l'indexation en rotation des deux parties **10**,**20** et visibles à la figure 5. Plus précisément, comme illustré aux figures 1 et 3, la deuxième partie **20** du dispositif comporte un corps principal **21**, de forme également cylindrique, dont le diamètre interne permet le passage de la partie haute du pulvérisateur, et en particulier de la collerette 5 de la pompe mécanique **3** Dans la partie basse, le corps **21** se prolonge par une portion 22 de plus fort diamètre, formant la zone de recouvrement avec la zone haute **17** de la première partie **10**. De préférence, le diamètre intérieur de la zone **22** et celui extérieur de la zone **17** sont ajustés pour permettre un coulissement avec un jeu quasi nul, assurant un guidage axial optimal. Tel qu'illustré à la figure 5, la face interne de la zone de recouvrement **22** présente une rainure **23** destinée à accueillir la nervure **16** présente sur la face extérieure de la première partie, afin d'assurer une indexation en rotation des deux parties **10**,**20** l'une par rapport à l'autre.

Pour revenir à la figure 1, on observe que la deuxième partie **20** comporte une ailette **25**, de forme analogue à l'ailette **13** de la première partie **10**. Cette allette **25** est disposée à proximité de l'extrémité haute de la partie **20**. Elle possède une courbure sensiblement analogue, et est équipée d'une nervure **26** permettant de renforcer sa tenue en flexion. Les largeurs des ailettes **25** peuvent être déterminées en fonction des efforts nécessaires pour assurer le fonctionnement de la pompe mécanique, et peuvent par exemple être configurées pour permettre l'accueil de deux doigts.

Tel que cela apparaît sur la figure 3, la deuxième partie **20** possède, à son niveau supérieur, une face **27** qui possède en son centre un évidement **28** destiné à laisser passer l'embout **7** du pulvérisateur. La partie périphérique de la zone **27** est destinée à venir en appui sur la surface **6** de la portion mobile **4** du pulvérisateur.

Pour assurer le blocage de la collerette **5** à l'intérieur du corps **20**, et comme illustré à la figure 6, des zones d'encliquetage ou analogue **30** peuvent être prévues au fond du corps de la deuxième partie **20**.

En pratique, différentes matières peuvent être utilisées pour réaliser le dispositif conforme à l'invention, parmi lesquelles on peut citer à titre d'exemple le polyéthylène, le polypropylène, mais également l'acrylonitrile butadiène styrène (ABS) ou le polycarbonate. Les matériaux choisis peuvent être formulés pour présenter une résistance aux pressions exercées, et posséder un coefficient de glissement propre à diminuer les forces de frottement qui s'exercent lors du coulissement des deux parties l'une par rapport à l'autre. Des considérations relatives au toucher et à l'esthétique peuvent également intervenir dans le choix des matériaux.

L'ensemble du dispositif d'aide peut être monté sur le pulvérisateur **1** de manière à donner la configuration illustrée à la figure 4.

Une pression ainsi exercée selon les flèches permet d'assurer un effort au niveau de la surface **6** de la collerette **5** et ainsi de mettre en oeuvre la pompe mécanique **3** Comme illustré à la figure 6, la course de coulissement entre la première et la deuxième partie **10**,**20** est supérieure à celle qui est nécessaire pour assurer le mouvement de la pompe mécanique **3**. On observe que la zone **32** située entre le haut de la deuxième partie **20** et la zone de coulissement **22**, à l'opposé de l'ailette **25**, est de superficie suffisante pour la mise en place d'une étiquette permettant d'identifier le contenu du pulvérisateur.

Dans une variante de réalisation illustrée à la figure 7, cette zone peut être évidée, de manière à laisser apparente une partie du pulvérisateur et en particulier du réservoir. Dans ce cas, le corps **121** de la deuxième partie **120**, occupe une partie suffisante de la circonférence du pulvérisateur **101** pour assurer un bon maintien mécanique. La partie évidée laisse ainsi apparaître la zone du pulvérisateur ou sont présentes les inscriptions d'identification du produit.

Des moyens complémentaires illustrés à la figure 8, telles que des nervures compressibles **112** ou des coques empilables, permettent de bloquer le pulvérisateur quelque soit son diamètre et ainsi d'utiliser le dispositif de l'invention avec différents diamètres de pulvérisateur. De même à diamètre constant, il est possible d'adapter le système à des récipients de capacité supérieure, et ainsi de couvrir une gamme allant par exemple de 5 à 30 ml simplement en modifiant la longueur du récipient et du système conforme à l'invention par une simple adaptation du moule industriel.

Il ressort de ce qui précède que le dispositif conforme à l'invention présente de multiples avantages, et en particulier une adaptation ergonomique permettant d'exercer une force non plus entre les premières phalanges du pouce et de l'index, mais entre les secondes phalanges de ces mêmes doigts, optimisant de ce fait la force à disposition. Il permet également de dégager l'embout de sortie et d'améliorer ainsi la visibilité de l'opération d'instillation.

## Revendications

1. Pulvérisateur équipé d'un dispositif d'assistance à la délivrance de gouttes d'un liquide contenu dans ledit pulvérisateur (1) comportant une portion mobile (4) formant une pompe mécanique, ladite portion mobile comportant une collerette (5) se prolongeant par un embout de sortie (7) présentant l'orifice (8) par lequel les gouttes sont délivrées ledit dispositif d'assistance comprenant :
- une première partie (10) agencée pour recevoir le fond du pulvérisateur (1) ;
- une deuxième partie (20) munie d'une face supérieure (27) possédant en son centre un évidemment (28) destiné à laisser passer l'embout (7) du pulvérisateur, la partie périphérique de la face (27) étant destinée à venir en appui sur la surface (6) de la collerette (5) de la portion mobile (4),
chaque partie (10,20) comportant une ailette d'appui latérale (13,25), les deux ailettes étant sensiblement en regard l'une de l'autre pour permettre un mouvement de la portion mobile (4) lorsqu'une force est exercée entre les deux ailettes (13,25).

2. Pulvérisateur selon la revendication 1, **caractérisé en ce que** les première (10) et deuxième (20) parties présentent une zone de recouvrement partiel (22) autorisant le coulissement relatif des deux parties (10,20) l'une par rapport à l'autre.

3. Pulvérisateur selon la revendication 2, **caractérisé en ce que** l'une et/ou l'autre partie (10,20) possède des excroissances (12) présentes à l'intérieur du corps recevant le pulvérisateur (1), de manière à bloquer le mouvement du pulvérisateur par rapport à l'une ou l'autre des parties.

4. Pulvérisateur selon la revendication 3, **caractérisé en ce que** les excroissances (12) sont compressibles.

5. Pulvérisateur selon la revendication 3, **caractérisé en ce que** la première partie (10) possède au moins une nervure (12) disposée dans une région du volume recevant le fond du pulvérisateur, et agencée pour venir au contact du pulvérisateur afin d'en bloquer le mouvement.

6. Pulvérisateur selon la revendication 1, **caractérisé en ce que** la seconde partie (20) présente des moyens (30) de solidarisation et en particulier d'encliquetage sur la partie mobile (4) du pulvérisateur.

7. Pulvérisateur selon la revendication 2, **caractérisé en ce qu'**il comporte, au niveau de la zone de recouvrement (22), des moyens d'indexation (16,23) des deux parties, afin de bloquer la rotation d'une partie (10) par rapport à l'autre (20).

8. Pulvérisateur selon la revendication 1, **caractérisé en ce qu'**au moins une des ailettes (13,25) présente une forme bombée dont la courbure est orientée vers l'ailette opposée.

9. Pulvérisateur selon la revendication 1, **caractérisé en ce que** l'une et/ou l'autre des ailettes (13,25) comporte au moins une nervure (14,26) reliant la face de l'ailette (13,25) orientée vers l'autre ailette et le corps de la partie (10,20) sur laquelle elle est implantée.

## Patentansprüche

1. Sprühgerät, das mit einer Hilfsvorrichtung zur Abgabe von Tropfen einer im Sprühgerät (1) enthaltenen Flüssigkeit ausgestattet ist, einen beweglichen Abschnitt (4) aufweisend, der eine mechanische Pumpe bildet, wobei der bewegliche Abschnitt einen Flansch (5) hat, der sich durch einem Austrittsansatz (7) verlängert, der die Öffnung (8) aufweist, durch welche die Tropfen abgegeben werden, wobei die Hilfsvorrichtung umfasst:
- ein erstes Teil (10), das zur Aufnahme des Bodens des Sprühgeräts (1) eingerichtet ist;
- ein zweites Teil (20), das mit einer Oberseite (27) ausgestattet ist, die in ihrer Mitte eine Ausnehmung (28) besitzt, die dazu bestimmt ist, den Ansatz (7) des Sprühgeräts durchtreten zu lassen, wobei das Umfangsteil der Seite (27) dazu bestimmt ist, an der Oberfläche (6) des Flanschs (5) des beweglichen Abschnitts (4) in Anlage zu kommen,
wobei jedes Teil (10, 20) einen seitlichen Anlagesteg (13, 25) hat, wobei die zwei Stege einander im Wesentlichen zugewandt sind, um eine Bewegung des beweglichen Abschnitts (4) zuzulassen, wenn eine Kraft auf die zwei Stege (13, 25) ausgeübt wird.

2. Sprühgerät nach Anspruch 1, **dadurch gekennzeichnet, dass** das erste (10) und zweite (20) Teil einen Teilüberdeckungsbereich (22) aufweisen, der die relative Verschiebung der zwei Teile (10, 20) in Bezug aufeinander erlaubt.

3. Sprühgerät nach Anspruch 2, **dadurch gekennzeichnet, dass** das eine und/oder das andere Teil (10, 20) Überstände (12) besitzt, die im Inneren des das Sprühgerät (1) aufnehmenden Körpers vorhanden sind, um die Bewegung des Sprühgeräts in Bezug auf das eine oder das andere Teil zu blockieren.

4. Sprühgerät nach Anspruch 3, **dadurch gekennzeichnet, dass** die Überstände (12) komprimierbar sind.

5. Sprühgerät nach Anspruch 3, **dadurch gekennzeichnet, dass** das erste Teil (10) mindestens eine Rippe (12) besitzt, die in einem Bereich des Volumens angeordnet ist, der den Boden des Sprühgeräts aufnimmt, und dazu eingerichtet ist, mit dem Sprühgerät in Kontakt zu kommen, um die Bewegung zu blockieren.

6. Sprühgerät nach Anspruch 1, **dadurch gekennzeichnet, dass** das zweite Teil (20) Einrichtungen (30) zur Befestigung und insbesondere zum Einrasten am beweglichen Abschnitt (4) des Sprühgeräts aufweist.

7. Sprühgerät nach Anspruch 2, **dadurch gekennzeichnet, dass** es im Bereich des Überdeckungsbereichs (22) Indexierungseinrichtungen (16, 23) für die zwei Teile hat, um die Drehung eines Teils (10) in Bezug auf das andere (20) zu blockieren.

8. Sprühgerät nach Anspruch 1, **dadurch gekennzeichnet, dass** mindestens einer der Stege (13, 25) eine gekrümmt Form aufweist, dessen Krümmung zum entgegengesetzten Steg gerichtet ist.

9. Sprühgerät nach Anspruch 1, **dadurch gekennzeichnet, dass** der eine und/oder der andere der Stege (13, 25) mindestens eine Rippe (14, 26) hat, welche die zum anderen Steg gerichtete Seite des Stegs (13, 25) und den Körper des Teils (10, 20) verbindet, an dem sie eingesetzt ist.

## Claims

1. Sprayer provided with a device that assists in delivering drops of a liquid contained in the said sprayer (1), comprising a movable portion (4) that forms a mechanical pump, with the said movable portion comprising a flange (5) that is extended by means of an outlet end piece (7) that has the opening (8) through which the drops are delivered, with the said assisting device comprising:
- a first part (10) arranged to receive the bottom of the sprayer (1);
- a second part (20) that has an upper surface (27) whose centre has a hole (28) that allows the sprayer's outlet end piece (7) to pass through, with the peripheral part of the surface (27) being meant to rest against the surface (6) of the flange (5) of the movable portion (4),
with each part (10, 20) including a side bearing fin (13, 25), the two fins being roughly opposite each other, so as to enable the movable portion (4) to move when a force is exerted between the two fins (13, 25).

2. Sprayer according to claim 1, **characterised in that** the first (10) and second (20) parts have an area of partial overlap (22) that enables the two parts (10, 20) to slide with respect to each other.

3. Sprayer according to claim 2, **characterised in that** one or both of the parts (10, 20) has/have outgrowths (12) within the body receiving the sprayer (1), such that they prevent the sprayer from moving with respect to either of the parts.

4. Sprayer according to claim 3, **characterised in that** the outgrowths (12) are compressible.

5. Sprayer according to claim 3, **characterised in that** the first part (10) has at least one rib (12) in a region of the volume that receives the bottom of the sprayer, and which is arranged so as to come into contact with the sprayer and restrict its movement.

6. Sprayer according to claim 1, **characterised in that** the second part (20) has means (30) to couple with, and, more specifically, to lock on to, the movable part (4) of the sprayer.

7. Sprayer according to claim 2, **characterised in that** it comprises, in the overlapping area (22), means (16, 23) to index both parts, so that one part (10) cannot rotate with respect to the other (20).

8. Sprayer according to claim 1, **characterised in that** at least one of the fins (13, 25) has a rounded shape whose curvature is oriented towards the opposite fin.

9. Sprayer according to claim 1, **characterised in that** one or both of the fins (13, 25) has/have at least one rib (14, 26) connecting the surface of the fin (13, 25) oriented towards the other fin and the body of the part (10, 20) on which it is installed.
